**Europäisches Patentamt**

**European Patent Office**

**Office européen des brevets**

(19)

(11) Numéro de publication: **0 214 136 B1**

(12)

# FASCICULE DE BREVET EUROPEEN

(45) Date de publication du fascicule du brevet:
**07.12.88**

(51) Int. Cl.⁴: **A 61 N 1/36,** A 61 H **39/00**

(21) Numéro de dépôt: **85902061.2**

(22) Date de dépôt: **30.04.85**

(86) Numéro de dépôt international:
**PCT/FR 85/00103**

(87) Numéro de publication internationale:
**WO 85/05042 (21.11.85** Gazette **85/25)**

(54) **APPAREIL DE TRAITEMENT MEDICAL POUR ETINCELLES APPLIQUEES SUR LA PEAU, A PARTIR D'ELECTRODES BIPOLAIRES.**

(30) Priorité: **04.05.84 FR 8407087**

(43) Date de publication de la demande:
**18.03.87 Bulletin 87/12**

(45) Mention de la délivrance du brevet:
**07.12.88 Bulletin 88/49**

(84) Etats contractants désignés:
**AT BE CH DE GB IT LI LU NL SE**

(56) Documents cités:
**DE-A- 2 117 917**
**FR-A- 2 500 745**
**FR-E- 65 169**

(73) Titulaire: **DERVIEUX, Dominique, Villa Lou Miou Rod Lieu dit des Cabanes, F-06790 Aspremont (FR)**

(72) Inventeur: **DERVIEUX, Dominique, Villa Lou Miou Rod Lieu dit des Cabanes, F-06790 Aspremont (FR)**

(74) Mandataire: **Hautier, Jean-Louis, OFFICE MEDITERRANEEN DE BREVETS D'INVENTION 24 rue Masséna, F-06000 Nice (FR)**

EP 0 214 136 B1

**Description**

L'invention a pour objet des électrodes bipolaires de décharge d'étincelles d'origine piézo-électrique pour le soulagement des douleurs et des contractures en application directe au contact de la peau.

Les électrodes actuellement utilisées sont des électrodes unipolaires. Le générateur piézo-électrique doit donc être mis à la masse. Cette disposition peut présenter quelques inconvénients: risque de décharge par mauvais contact avec l'utilisateur au niveau de la mise à la masse; risque de décharge lorsqu'un tiers, qui utilise l'appareil pour traiter le malade, entre en contact avec lui; peu d'efficacité sur les douleurs et les contractures...

Ces appareils sont, en général, utilisés pour l'acupuncture. Avant de faire agir les électrodes, il est nécessaire de repérer les points d'acupuncture adéquats.

L'état de la technique peut-être défini par les brevets suivants:

— FR-A-2 500 745 au nom de DERVIEUX, ce brevet décrit un appareil de soulagement des douleurs par stimulation de points d'acupuncture utilisant des décharges d'origine piézo-électrique à l'aide d'une électrode UNIPOLAIRE. L'appareil comprend un générateur piézo-électrique muni d'un moyen faisant office de poignée d'action de l'appareil et d'au moins une électrode reliée au générateur.

— FR-E-65 169, ce brevet décrit une électrode bipolaire dont les deux pôles sont maintenus à distance l'un par rapport à l'autre mais en aucun cas maintenus à distance par rapport à la peau par un moyen de séparation isolant.

L'invention tend à résoudre ces problèmes techniques et, dans son application, vise notamment des effets antalgiques (anti-douleureux) et décontractants, tant chez l'homme que chez l'animal.

Les électrodes selon l'invention utilisent, de manière caractéristique, du courant piézo-électrique. Ledit courant piézo-électrique est utilisé pour la décharge d'étincelles. Le courant piézo-électrique peut être de trois mille volts en pics (environ un à dix mille volts) de quelques dizaines de milli-seconde, de cinq centièmes de milli-ampère à la pression et d'un milli-ampère au relachement de la poignée de manoeuvre du générateur piézo-électrique entre les deux pôles de l'électrode. Le courant est positif sur un des pôles de l'électrode et négatif sur l'autre pôle de l'électrode par des décharges simultanées. Il y a inversion de la polarisation entre la pression et le relachement.

Les électrodes selon l'invention sont également caractéristiques.

Les électrodes sont bipolaires. Un pôle est relié à la sortie du générateur piézo-électrique (fil isolé de sortie), l'autre pôle est relié au cadre du générateur piézo-électrique (sortie en contact avec l'autre pôle du ou des cristaux).

Les électrodes, en matière conductrice d'électricité, sont formées de pointes de décharge d'étincelles, soit des pointes fixes sur un support (pointes en dents de scie sur une lame, reliefs sur tout support, brosse...) d'une à plusieurs dizaines par pôles, soit des pointes séparées, soit d'une simple plaque, lame ou barre...

Les pôles desdites électrodes sont espacés de moins d'un centimètre (en général de quelques centimètres au plus) de manière à éviter la sensation de double décharge et pour apporter une meilleure stimulation.

Un moyen de séparation est disposé entre les deux pôles des électrodes.

Ledit moyen de séparation est plus long de quelques dixièmes de millimètre que la longueur des pointes des électrodes de manière à ce que les électrodes soient à quelques dixièmes de millimètre de la peau lorsque l'appareil est appliqué en contact contre la peau.

Ledit moyen de séparation est en matière isolante pour éviter l'interdécharge des pôles.

Ledit moyen de séparation peut s'évaser en laissant dépasser de l'orifice de l'évasement, environ un millimètre du pôle (ou d'une partie d'un pôle de décharge, par exemple: une pointe) et permettre ainsi un cône de décharges possibles.

Une autre carctéristique des électrodes réside dans leur forme qui leur assure une spécificité particulière:

— électrodes à lames à plusieurs pointes ou à aiguilles; il y a deux lames ou deux séries d'aiguilles parallèles, séparées par un moyen de séparation isolant,

— électrodes en couronne à plusieurs pointes; il y a un pôle central constitué d'une ou de plusieurs pointes ou même d'une couronne, une couronne de séparation isolante et une couronne extérieure concentrique.à plusieurs pointes.

Les électrodes à lames à plusieurs pointes ou séries d'aiguilles parallèles ont une action antalgique et réflexe.

Les électrodes en couronne à plusieurs pointes ont une action décontracturante et trophique.

Les dessins ci-joints donnés à titre d'exemple indicatifs et non limitatifs permettront aisément de comprendre l'invention. Ils représentent des modes de réalisations préférés selon l'invention.

La figure 1 est une vue de côté, dans sa longueur et d'en haut, de l'electrode bipolaire à aiguilles, sans les séparations.

La figure 2 est une vue de côté de l'électrode bipolaire à aiguilles, vue en coupe transversale au niveau de deux aiguilles (une de chacun des pôles de l'électrode).

La figure 3 est une vue de face de l'électrode bipolaire à aiguilles.

La figure 4 est une vue de côté, dans sa longueur et d'en haut, d'une électrode bipolaire à lame à pointes en dents de scie.

La figure 5 est une vue en coupe transversale d'une électrode bipolaire à lame à pointes en dents de scie.

La figure 6 est une vue de face d'une électrode bipolaire à lame à pointes en dents de scie.

La figure 7 est une vue d'une électrode bipolaire, vue de face.

La figure 8 est une vue d'une électrode bipolaire vue en coupe transversale.

La figure 9 est une vue d'une électrode bipolaire vue en coupe transversale, laissant apparaître le pôle externe en «couronne».

La figure 10 est une vue du branchement du corps du générateur piézo-électrique, vue de face.

La figure 11 est une vue de l'électrode à lames à plusieurs pointes en dents de scie, vue de face.

La figure 12 est une vue de l'électrode à lames à plusieurs pointes en dents de scie, vue postérieure.

La figure 13 est une vue de l'électrode à lames à plusieurs pointes en dents de scie, vue en coupe selon l'axe longitudinal d'une des deux lames.

La figure 14 est une vue de l'électrode à lames à plusieurs pointes en dents de scie, vue en coupe selon l'axe transversal des lames.

La figure 15 est une vue de l'électrode bipolaire en couronne, vue de face.

La figure 16 est une vue de l'électrode bipolaire en couronne, vue postérieure.

La figure 17 est une vue de l'électrode bipolaire en couronne, vue en coupe longitudinale.

La figure 18 est une vue de l'électrode bipolaire en couronne, vue des deux pôles de l'électrode en couronne.

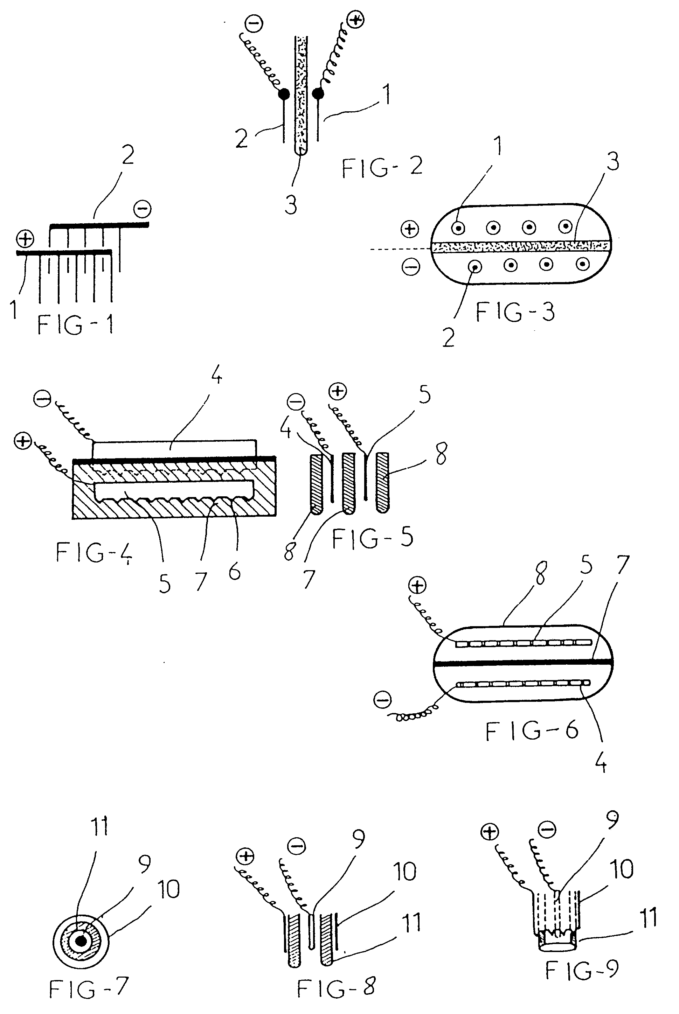

Dans les figures 1, 2 et 3, qui représentent une électrode bipolaire à aiguilles dont la forme est à visée antalgique et réflexe, les électrodes 1 et 2 sont disposées selon deux plans parallèles.

Un moyen de séparation isolant 3 est disposé entre lesdites électrodes 1 et 2.

Cette cloison de séparation 3, en matière isolante, est plus longue de quelques dixièmes de millimètre que la longueur des pôles de l'électrode 1 et 2. Cette cloison de séparation 3 vient en contact avec la peau et permet ainsi d'éviter l'interdécharge des pôles. Cette cloison de séparation permet la mise à distance des pôles par rapport à la peau: environ deux à trois dixièmes de millimètre (de quelques dixièmes de millimètre à une dizaine de millimètres).

Dans les figures 4, 5 et 6, sont représentées des électrodes bipolaires 4, 5 à lame à pointes 6 en dents de scie, dont la forme est également à visée antalgique. Bien entendu, il est possible de concevoir pour chaque pôle de l'électrode une ou plusieurs lames à pointes 6 en dents de scie.

La cloison de séparation 7, en matière isolante, est disposée impérativement entre les deux lames des électrodes 4, 5 dans un plan parallèle. Cette cloison de séparation 7 peut également entourer l'ensemble de la tête du dispositif par une cloison 8 et appartenir ainsi à tout le corps de l'électrode en une seule pièce.

Les figures 7, 8 et 9 représentent une électrode bipolaire 9, 10 en couronne à pointes en dents de scie dont la forme spécifique en couronne est à visée décontracturante et trophique.

Le pôle central 9 de l'électrode est entouré d'une cloison de séparation en matière isolante 11, elle-même entourée d'une autre couronne coaxiale qui est l'autre pôle 10 de l'électrode en couronne.

La cloison de séparation 11, en matière isolante, est plus longue de quelques millimètres que les pôles de l'électrode 9 et 10. Cette cloison de séparation 11 vient en contact avec la peau et permet ainsi d'éviter l'interdécharge des pôles. Cette cloison de séparation permet la mise à distance des pôles par rapport à la peau, environ deux à trois dixièmes de millimètre (de quelques dixièmes de millimètre à une dizaine de millimètre) comme pour l'électrode à lames parallèles.

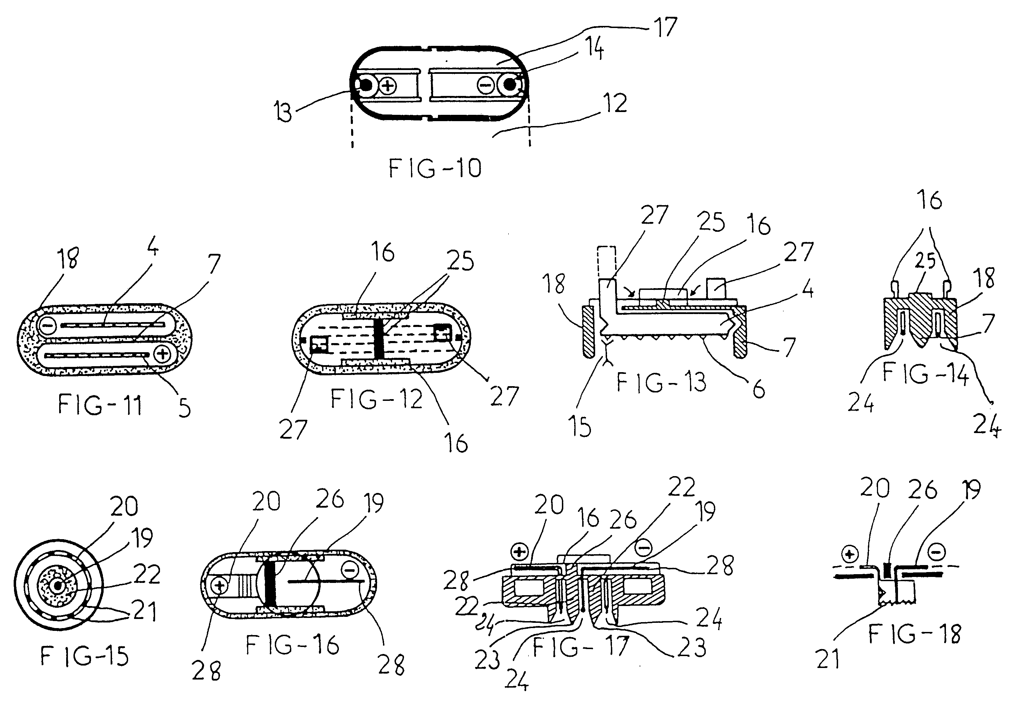

La figure 10 est une vue du branchement du corps 12 du générateur piézo-électrique, avec une vue du pôle positif 13 et du pôle négatif 14, leur éloignement évite toute décharge entre-eux. Un pôle est relié à la sortie du générateur piézo-électrique (fil isolé de sortie), l'autre pôle est relié au cadre du générateur piézo-électrique (sortie en contact avec l'autre pôle du ou des cristaux). Il y a inversion de polarisation entre la pression et le relâchement du levier de ce générateur.

Les figures 11, 12, 13 et 14 représentent une électrode à lames 4, 5 à pointes 6 en dents de scie. Il y a lieu de noter la distance 15 qui existe entre la longueur de la cloison de séparation isolante 7 et les pointes 6 des pôles 4, 5 de l'électrode à lames.

Des moyens de fixation amovibles 16 permettent de fixer et de maintenir branchées les électrodes sur la tête de réception 17 du corps du générateur piézo-électrique 12.

Les pointes 6 des dents des lames de l'électrode dépassent d'environ un millimètre l'orifice d'évasement de la cloison de séparation 7 et du corps isolant 18 de l'électrode, au niveau d'une fente s'évasant pour permettre un cône de décharge 24.

Les figures 12, 13 et 14 montrent la séparation 25 qui évite une décharge entre les pôles 4 et 5 au niveau de leur petite lame de contact postérieur 27 qui est recourbée. Ces petites lames de contact postérieur 27 permettent le branchement de l'électrode avec les deux sorties 13, 14 du générateur piézo-électrique. Tous types de branchement peuvent être réalisés entre les électrodes et les sorties du générateur piézo-électrique mais à condition d'une parfaite isolation.

Les figures 15, 16, 17 et 18 représentent une électrode bipolaire à deux pôles 19, 20 (en couronne à pointes en dents de scie).

Dans un mode de réalisation représenté, il y a huit pointes 21. Dans ce mode de réalisation, c'est l'extrémité 23 des cloisons 22 en matière isolante qui fait office de cône de décharge.

Les figures 16, 17 et 18 montrent la séparation 26 qui évite une décharge entre les pôles 19, 20, ici très rapprochés au niveau de leur sortie postérieure qui est recourbée pour faire contact à leur extrémité 28 avec les deux sorties 13, 14 du générateur piézo-électrique.

L'invention a pour objet:

Des électrodes bipolaires spécifiques: à pôles rapprochés et nécessitant une isolation particulière produisant une décharge d'étincelles d'origine piézo-électrique pour le soulagement des douleurs et des contractures.

Les électrodes *bipolaires* pour le *soulagement des douleurs et la stimulation des zones réflexes*:

— elle utilisent un mode d'action neurophysiologique: la Neuro-Stimulation Trans-Cutanée (NSTC), électrostimulation périphérique exerçant une action inhibitrice sur les cellules de relais médullaires activées par des flux nociceptifs (stimulation des fibres nerveuses sensitives de gros diamètre: A, α, β) ainsi qui le déclenchement d'une sécrétion d'endorphines.

L'électrode type peut être défini ainsi:

— au moins deux lames (ou deux séries de plusieurs aiguilles) munies de plusieurs pointes (optimum huit) en dents de scie;

— ces pointes (ou aiguilles) seront décalées d'un pôle de l'électrode par rapport à l'autre pour permettre un meilleur balayage (un pic de décharge tous les un à dix millimètres en moyenne);

— elles seront branchées séparément sur les deux sorties du générateur piézo-électrique (fil de sortie et corps du générateur);

— elles seront séparées par une cloison de matière isolante (plastique ou autre) qui ira au contact de la peau (pour éviter une décharge entre les deux pôles);

— cette cloison de séparation, et éventuellement le corps de l'électrode, permettront de conserver la distance des pointes de décharges des deux pôles entre un et dix millimètres environ (deux à trois millimètrs en moyenne), cette cloison de séparation et éventuellement le corps neutre de l'électrode étant mis en contact direct avec la peau lors de l'utilisation de l'électrode.

Les pointes des deux pôles apparaîtront et déborderont d'un millimètre dans un cône ou une fente s'évasant, pour permettre un cône de décharge. Cet évasement est prévu entre les différentes séparations et le corps de l'électrode.

Le maniement de l'appareil muni des électrodes selon l'invention est le suivant:

— Criblage d'étincelles piézo-électriques, systématique, d'une zone douloureuse, ou projetée, ou rapportée, ou d'un dermatome réflexe, en débordant largement cette zone (cinq à dix centimètres autour) durant quelques secondes à plusieurs minutes (optimum: vingt à quarante secondes) par déplacement (balayage) permanent de l'électrode (sans jamais rester fixe) dans toutes les sens, sans systématisation.

Les électrodes *bipolaires* à action *décontracturante et trophique*:

un autre but de l'invention est de stimuler des «points moteurs» musculaires au travers de la peau par l'électrode «en couronne» bipolaire pour un effet décontracturant et trophique.

On utilise la régulation du tonus et la suppression des contractures par la boucle «γ» par décharges piézo-électriques au niveau des «plaques neuro-motrices» ou «points moteurs». De même, on obtiendra un effet trophique par cette même stimulation.

Description de l'électrode type à action décontracturante et trophique:

— une pointe métallique centrale (arrondie ou aiguë) reliée à l'un des branchements;

— une «couronne» métallique périphérique circulaire à pointes en dents de scie (deux à dix; huit optimum; on peut utiliser la même pièce que pour les pôles de l'électrode «antalgique» mais roulée cylindriquement) reliée à l'autre branchement électrique;

— un moyen de séparation cylindrique coaxial, en matière isolante, doit être mis au contact de la peau pour éviter la décharge entre les pôles de l'électrode et permettre une mise à distance des pôles de deux à quatre dixièmes de millimètre environ (trois dixièmes de millimètre optimum; deux dixièmes de millimètre pour la «couronne» et trois dixièmes de millimètre pour l'électrode centrale permettraient une décharge accrue);

— les pointes des deux pôles apparaîtront, sur un millimètre environ, à l'orifice d'un cône ou d'une fente évasée pour permettre un cône de décharge.

Le maniement de l'appareil muni desdites électrodes est le suivant:

On stimule des zones réflexes ou des muscles par décharges piézo-électriques, au niveau des «points moteurs», par un léger mouvement de bascule circulaire autour de l'axe de l'électrode sans déplacement de celle-ci, durant quelques secondes à quelques dizaines de secondes (rarement plus de vingt), uniquement lorsque l'on est au niveau du «point moteur» que l'on aura eu soin de rechercher par quelques stimulations en déplaçant l'électrode.

L'appareil complet comprendra un corps contenant le générateur piézo-électrique et pourra recevoir, au niveau de sa tête, une électrode fixe ou plusieurs électrodes bipolaires amovibles selon les moyens de fixation 16.

L'électrode sera donc déplacée avec le corps de l'appareil, celui-ci sera muni d'un levier d'actionnement du générateur piézo-électrique, totalement isolé électriquement.

**Revendications**

1. Electrode pour un appareil de soulagement des douleurs et des contractures en application directe à la peau, comprenant un générateur piézo-électrique muni d'un moyen d'actionnement placé dans la poignée de l'appareil et d'au moins une électrode reliée au dit générateur, caractérisée par le fait qu'il s'agit d'une électrode bipolaire dont les deux pôles sont maintenus à distance du plan d'appui de l'électrode sur la peau.

2. Electrode bipolaire selon la revendication 1, caractérisée par le fait que le moyen de maintien à distance du plan d'appui des pôles de d'echarge de l'électrode sur la peau est une cloison de séparation en matière isolante plus longue que les pôles de décharge de l'électrode.

3. Electrode bipolaire selon l'une des revendications 1 ou 2, caractérisée par le fait que la cloison de séparation fait partie du corps isolant entourant l'électrode par l'extérieur, tandis que le bord de ce corps de la cloison finissent dans le même plan d'appui sur la peau et sont plus longs que l'extrémité des pôles de décharge de l'électrode.

4. Electrode bipolaire selon la revendication 3, caractérisée par le fait que la cloison et le corps font office de cône de décharge pour chaque pôle, laissant dépasser l'extrémité des pôles de décharge de l'électrode dans l'orifice d'evasement du cône.

5. Electrode bipolaire selon l'une quelconque des revendications 1 à 4, caractérisée par le fait que chacun des pôles est constitué d'une série de pointes ou d'aiguilles de décharge, disposées parallèlement à l'autre et entre lesquelles est disposée la cloison de séparation en matière isolante.

6. Electrode bipolaire selon l'une quelconque des revendications 1 à 4, caractérisée par le fait que chacun des pôles de décharge est constitué d'une lame, barre ou plaque, disposée parallèlement à l'autre et entre lesquels est disposée la cloison de séparation en matière isolante.

7. Electrode bipolaire selon la revendication 6, caractérisée par le fait que la lame, barre ou plaque comporte des pointes de décharge en dents de scie.

8. Electrode bipolaire selon la revendication 5 ou 7, caractérisée par le fait que les pointes des aiguilles ou des dents de scie de chacun des pôles de décharge sont espacées d'un à dix millimètres et décalées d'un pôle à l'autre.

9. Electrode bipolaire selon l'une quelconque des revendications 1 à 4, caractérisée par le fait que l'électrode est constituée d'un pôle central de décharge entouré d'une cloison cylindrique en matière isolante, entournée elle-même par l'autre pôle de décharge.

10. Electrode bipolaire selon la revendication 9, caractérisée par le fait que le pôle extérieur comporte plusieurs pointes de décharge.

11. Electrode bipolaire selon l'une quelconque des revendications 1 à 10, caractérisée par le fait qu'elle est réalisée démontable et comporte des moyens de fixation et de connection sur la tête de la poignée pouvant contenir le générateur piézo-électrique, par des contacts espacés et isolés.


**Patentansprüche**

1. Elektrode für ein Gerät zur Schmerzbefreiung und zum Lösen von Krämpfen durch direkte Anwendung auf der Haut, mit einem piezo-elektrischen Generator, der mit einem Betätigungsorgan versehen ist, das im Griff des Gerätes angeordnet ist und mindestens mit einer an den Generator angeschlossenen Elektrode, dadurch gekennzeichnet, dass es sich um eine bipolare Elektrode handelt, dessen beiden Pole von der Auflageebene der Elektrode auf der Haut beabstandet gehalten werden.

2. Bipolare Elektrode nach Anspruch 1, dadurch gekennzeichnet, dass das Organ zum Beabstanden der Entladungspole der Elektrode von der Auflageebene auf der Haut eine Trennwand aus isolierendem Material ist, die länger ist als die Pole der Entladungselektrode.

3. Bipolare Elektrode nach einem der Ansprüche 1 oder 2, dadurch gekennzeichnet, dass die Trennwand Teil des Isolierkörpers ist, der die Elektrode aussen umhüllt, während der Rand des Körpers und der Trennwand in derselben Auflageebene auf der Haut enden und länger sind als die Enden der Entladungspole der Elektrode.

4. Bipolare Elektrode nach Anspruch 3, dadurch gekennzeichnet, dass die Trennwand und der Körper einen Entladungskonus für jeden Pol bilden, wobei das Ende eines jeden Entladungspols in der Ausweitung des Konus mündet.

5. Bipolare Elektrode nach einem der Ansprüche 1 bis 4, dadurch gekennzeichnet, dass jeder Pol aus einer Reihe von Entladungspunkten oder Entladungsnadeln besteht, die parallel zueinander angeordnet sind und zwischen denen eine Trennwand aus Isoliermaterial angeordnet ist.

6. Bipolare Elektrode nach einem der Ansprüche 1 bis 4, dadurch gekennzeichnet, dass jeder der Entladungspole aus einem Blatt, einer Stange oder einer Platte besteht, die parallel zueinander angeordnet sind und zwischen denen die Trennwand aus Isoliermaterial angeordnet ist.

7. Bipolare Elektrode nach Anspruch 6, dadurch gekennzeichnet, dass das Blatt, die Stange oder die Platte sägezahnförmige Entladungspunkte aufweisen.

8. Bipolare Elektrode nach Anspruch 5 oder 7, dadurch gekennzeichnet, dass die Spitzen der Nadeln oder der Sägezähne eines jeden Entladungspols mit einem Abstand von einem bis 10 mm und versetzt zueinander angeordnet sind.

9. Bipolare Elektrode nach einem der Ansprüche 1 bis 4, dadurch gekennzeichnet, dass die Elektrode aus einem mittleren Entladungspol besteht, der von einer zylinderförmigen Wandung aus Isoliermaterial umgeben ist, die wiederum von dem anderen Entladungspol umgeben wird.

10. Bipolare Elektrode nach Anspruch 9, dadurch gekennzeichnet, dass der äussere Pol mehrere Entladungspunkte aufweist.

11. Bipolare Elektrode nach einem der Ansprüche 1 bis 10, dadurch gekennzeichnet, dass sie abmontierbar ausgebildet ist und Befestigungsorgane und Verbindungsorgane zur Verbindung am Kopf des Griffes aufweist, der den piezo-elektrischen Generator aufweisen kann, wobei Verbindung durch beabstandete und voneinander isolierte Kontakte hergestellt wird.


**Claims**

1. Electrode for pain-killing and antispasmodic apparatus for direct application on the skin, comprising a piezo-electric generator having activation means arranged within the handle of said apparatus and at least one electrode connected to said generator, characterized in that said electrode is a bipolar electrode the two poles of which are arranged distant from the plane of contact of said electrode with the skin.

2. Bipolar electrode according to claim 1, characterized in that said means for keeping distant said decharging poles of said electrode from the plane of contact with the skin is a partition wall made of insulating material and being longer than said discharge poles of the electrode.

3. Bipolar electrode according to one of claims 1 or 2, characterized in that said partition wall is a part of an insulating body covering said electrode at its outer surface, the border of said body and said partition wall ending within the same plane of contact with the skin and being longer than the tips of said poles of the decharge electrodes.

4. Bipolar electrode according to claim 3, characterized in that said partition wall and said body serve as a decharge cone for each pole, the tip of each decharge poles of said electrode passing through the evasion-orifice of said cone.

5. Bipolar electrode according to one of claims 1 to 4, characterized in that each pole consists of a series of decharge-points or -needles, being disposed in parallel to each other a partition wall made of insulating material being disposed between them.

6. Bipolar electrode according to one of claims 1 to 4, characterized in that each decharge pole consists of a blade, a bar or a plate, disposed in parallel to each other and the partition wall made of insulating material being disposed between them.

7. Bipolar electrode according to claim 6, characterized in that the blade, the bar or the plate comprise decharge points in form of saw-teeth.

8. Bipolar electrode according to claim 5 or 7, characterized in that said needle tips or said saw-teeth of each of said poles are spaced to each other with a distance of 1 to 10 millimeters and are shifted with respect to each other.

9. Bipolar electrode according to one of claims 1 to 4, characterized in that the electrode consists of a central decharge pole covered with a cylindrical partition wall made of insulating material which itself is surrounded by the other decharge pole.

10. Bipolar electrode according to claim 9, characterized in that the exterieur pole comprises a plurality of decharge points.

11. Bipolar electrode according to one of claims 1 to 10, characterized in that it is realized demontably and in that it comprises fixation means and connection means to connect it on the head of said handle, which may comprise the piezo-electrical generator, by spaced isolated contacts.

FIG-1

FIG-2

FIG-3

FIG-4

FIG-5

FIG-6

FIG-7

FIG-8

FIG-9

FIG-10

FIG-11

FIG-12

FIG-13

FIG-14

FIG-15

FIG-16

FIG-17

FIG-18